(19) Europäisches Patentamt
European Patent Office
Office européen des brevets

(11) **EP 1 129 730 B2**

(12) **NEW EUROPEAN PATENT SPECIFICATION**
After opposition procedure

(45) Date of publication and mention
of the opposition decision:
**02.01.2013   Bulletin 2013/01**

(45) Mention of the grant of the patent:
**14.07.2010   Bulletin 2010/28**

(21) Application number: **99954374.7**

(22) Date of filing: **04.11.1999**

(51) Int Cl.:
**A61L 15/28** *(2006.01)*      **A61L 15/60** *(2006.01)*
**A61F 13/537** *(2006.01)*

(86) International application number:
**PCT/JP1999/006141**

(87) International publication number:
**WO 2000/027443 (18.05.2000 Gazette 2000/20)**

(54) **PRODUCTS FOR THICKENING PROCESSING BODILY FLUIDS OR BODILY WASTES**

PRODUKTE ZUM EINDICKEN VON KÖRPERFLÜSSIGKEITEN ODER KÖRPERAUSSCHEIDUNGEN

PRODUITS POUR EPAISSIR DES FLUIDES OU DES DECHETS CORPORELS

(84) Designated Contracting States:
**DE FR GB**

(30) Priority: **09.11.1998   JP 31792398**

(43) Date of publication of application:
**05.09.2001   Bulletin 2001/36**

(73) Proprietor: **KAO CORPORATION**
**Tokyo 103-0025 (JP)**

(72) Inventors:
• **Kasai, Takao,**
**Kao Corporation/Research Lab.**
**Haga-gun,**
**Tochigi 321-3426 (JP)**
• **Minowa, Hiroki,**
**Kao Corporation/Research Lab.**
**Haga-gun,**
**Tochigi 321-3426 (JP)**
• **Ito, Taketo,**
**Kao Corporation/Research Lab.**
**Haga-gun,**
**Tochigi 321-3426 (JP)**

(74) Representative: **Vossius & Partner**
**Siebertstrasse 4**
**81675 München (DE)**

(56) References cited:
| | |
|---|---|
| **JP-A- 1 221 575** | **JP-A- 56 097 450** |
| **JP-A- 60 104 503** | **US-A- 3 070 095** |
| **US-A- 3 347 236** | **US-A- 4 381 784** |
| **US-A- 5 126 382** | **US-A- 5 721 295** |

## Description

Technical Field:

The present invention relates to an article for thickening body fluids or excreta such as disposable diapers, sanitary napkins, liners for diapers, panty liners and surgical blood absorbent articles, etc. More particularly the present invention relates to an article capable of thickening body fluids or excreta which shows high leakproofing effect against high viscous liquids such as soft feces or blood.

Background Art:

Absorbent articles such as disposable diapers and sanitary napkins comprises an absorbent member for absorbing waste which has particles of a superabsorbent polymer such as a crosslinked polyacrylic acid salt, dispersed in pulp fiber.

The superabsorbent polymer does not move in the absorbent member. It is not until liquid reaches the position where the polymer exists that the polymer absorbs the liquid and fixes it. Accordingly, when a high viscous liquid having a small rate of permeation and diffusion, such as soft feces or blood, is discharged, it is likely that not all the liquid can arrive at the position where the superabsorbent polymer is, or it takes time for all the liquid to reach the position and be absorbed and fixed thereby. Meanwhile a leak can result. It is a conceivable solution to dispose the superabsorbent polymer near the wearer facing surface of the absorbent article thereby reducing the time required for the liquid to reach. In this case, however, the superabsorbent polymer tends to cause a gel blocking phenomenon which interferes with absorption.

An absorbent article using a cross-linkable swellable polysaccharide as an absorbent in place of a superabsorbent polymer is proposed in WO 95/17147. In this absorbent article, the polysaccharide is used in the form of particles which are bound to the surface of a solid substance or incorporated into the substrate matrix so that it may absorb liquid even under pressure and exhibit improved liquid retentivity for an extended period of time. Similarly to the superabsorbent polymer, the bound or incorporated polysaccharide itself does not diffuse in liquid waste but waits for liquid to come. It does not display absorbing and fixing effects until liquid reaches. Therefore, the above-mentioned absorbent article does not have sufficient leakproofness against high viscous liquids similarly to the conventional ones using superabsorbent articles.

Disclosure of the Invention:

Accordingly, an object of the present invention is to provide an article capable of thickening body fluids or excreta which is highly effective in preventing leaks particularly of a high viscous liquid, such as soft feces or blood.

Another object of the present invention is to provide an article capable of thickening body fluids or excreta which is particularly useful as a disposable diaper or a liner for a diaper worn by newborn babies or infants who get rid of soft feces frequently, a sanitary napkin or a panty liner.

The inventors of the present invention have found that the above objects are accomplished by incorporating a specific polysaccharide in a specific state and utilizing the thickening effect of the polysaccharide.

The present invention has been completed based on the above finding. The present invention accomplishes the above objects by providing an article for thickening body fluids or excreta comprising a liquid retentive absorbent member (4) which has a double-layered structure having an upper absorbent member (4a) and a lower absorbent member (4b) wherein a polysaccharide powder (10) capable of thickening in the presence of a polyvalent metal ion is present in the upper absorbent member (4a), in a state ready to dissolve or be dissociated in the moisture contained in body fluids or excreta, wherein said polysaccharide (10) contains no polyvalent metal ions, and wherein the lower absorbent member (4b) comprises pulp fiber (9) having superabsorbent polymer particles (12) dispersed therein or wherein the lower absorbent member (4b) consists solely of the superabsorbent polymer particles (12), wherein "upper" means facing to the wearer and wherein the upper absorbent member (4a) predominantly comprises pulp fiber (9) and the polysaccharide (10) is dispersed uniformly throughout the upper absorbent member (4a) in planar and thickness directions and they are wrapped in absorbent paper (11) to form the upper absorbent member (4a).

Brief Description of the Drawings:

Fig. 1 is a plan view of a disposable diaper as an embodiment of the thickening article of the present invention with a part cut away, seen from its topsheet side.

Fig. 2 is an enlarged cross-section of Fig. 1 taken along line I-I.

Best Mode for Carrying out the Invention:

A preferred embodiment of the article for thickening body fluids or excreta (hereinafter simply referred to as a thickening article) according to the present invention will be described below with reference to a disposable diaper as an example and to the accompanying drawings.

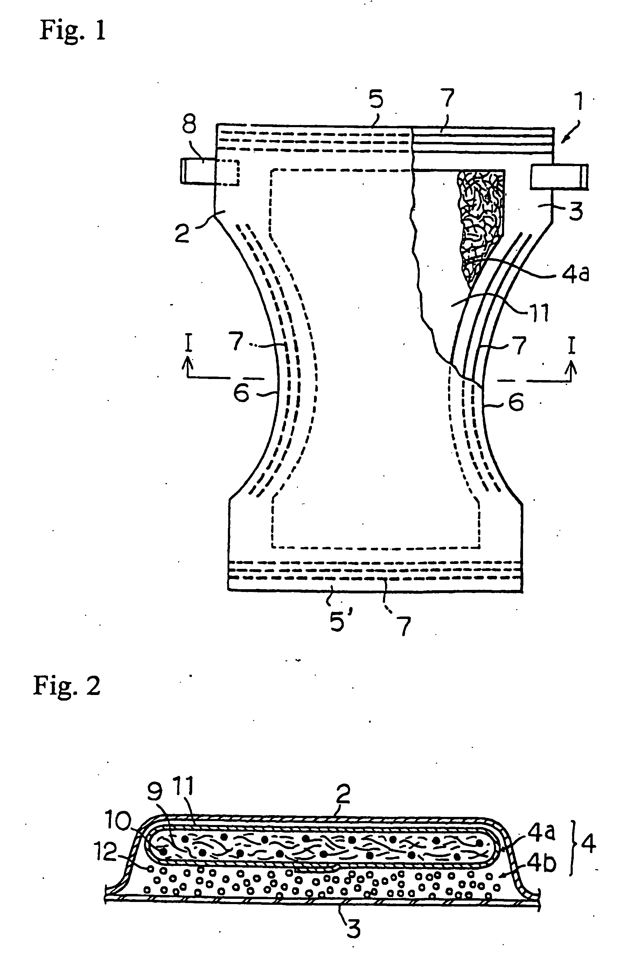

As shown in Figs. 1 and 2, the disposable diaper 1 of the embodiment comprises a liquid permeable topsheet 2 which is made of nonwoven fabric, a perforated film, etc.; a liquid impermeable backsheet 3 which serves as a leakproof layer; and a liquid retentive absorbent member 4 which is interposed between the topsheet 2 and the backsheet 3 as an absorbing layer. The absorbent member 4 has its portion corresponding to the crotch portion of the diaper 1 curved inward to form a sandglass shape. The topsheet 2 and the backsheet 3 also have their portion corresponding to the crotch portion of the diaper 1 curved inward in conformity to the shape of the absorbent member 4. The absorbent member 4 is held and fixed between the topsheet 2 and the backsheet 3.

The topsheet 2 and the backsheet 3 extend outward from the front and the rear ends and the lateral sides of the absorbent member 4 to form a front and a rear waist portion 5' and 5 and a pair of leg portions 6 and 6. The front and the rear waist portions 5' and 5 and the pair of leg portions 6 and 6 are each provided with elastically stretchable members 7 for giving the waist portions 5 and 5' and the leg portions 6 and 6 a good fit to a wearer's body when put on the wearer. The stretchable members 7 are fixed by the topsheet 2 and the backsheet 3. A pair of fastening members 8, such as sets of tape fasteners, are attached to both lateral sides of the rear waist portion 5. While not shown, a mating member, such as a rectangular landing tape, is provided on the backsheet 3 in the front waist portion 5'. On putting the disposable diaper 1 of the embodiment on a wearer, the fastening members 8 are stuck on this mating member. The above-mentioned constitution and structure are the same as in conventional disposable diapers.

In the disposable diaper 1 according to this embodiment, the absorbent member 4 has a double-layered structure having an upper absorbent member 4a and a lower absorbent member 4b as shown in Fig. 2. The upper absorbent member 4a predominantly comprises pulp fiber 9. A polysaccharide powder 10 capable of thickening in the presence of a polyvalent metal ion is dispersed uniformly throughout the upper absorbent member 4a in planar and thickness directions. They are wrapped in thin absorbent paper 11, e.g., tissue, to form the upper absorbent member 4a. The lower absorbent member 4b comprises pulp fiber (not shown) having superabsorbent polymer particles 12 dispersed and mixed uniformly. As will be obvious from Examples given later, the lower absorbent member 4b can consist solely of the superabsorbent polymer particles 12.

As a result of the present inventors' study, it has been revealed that incorporation of the above-described polysaccharide into the disposable diaper 1 brings about an enhanced effect against leaks of a highly viscous liquid, such as soft feces or blood. The reasons for this effect enhancement are assumed to be as follows. As previously noted, a superabsorbent polymer used in an absorbent member of conventional disposable diapers, sanitary napkins, etc. does not migrate in the absorbent member and cannot exert its absorbing and fixing performance until a liquid comes to. That is, its absorption and fixation mechanism is, so to speak, passive. On the other hand, the polysaccharide exhibits a liquid fixing effect through gelation similarly to the superabsorbent polymer. Additionally, the polysaccharide is capable of dissolving or being dissociated in water and diffusing in water. So, once liquid waste and the polysaccharide meet, the part of the liquid that is in contact with the polysaccharide is fixed, while the polysaccharide diffuses in the liquid through dissolution or dissociation so that the part of the liquid that is distant from the polysaccharide can also be fixed. In other words, the polysaccharide has, so to speak, a positive mechanism of absorption and fixation. Therefore, even when a high viscous liquid cannot completely get to the position where the polysaccharide is, the polysaccharide is capable of fixing the high viscous liquid. Besides, in the present invention the polysaccharide reacts with trace amounts of polyvalent metal ions present in body fluids or excreta (e.g., calcium ions and aluminum ions) to form complexes having increased viscosity, which come into contact with liquid waste and, as a result, thicken the liquid to develop a fixing effect. Such properties of the polysaccharide enhance the leakproofing effect against high viscous liquids such as soft feces and blood as well as low viscous liquids such as urine. Additionally, since the polysaccharide does not cause gel blocking, it is possible to dispose it near the wearer facing surface of a disposable diaper, a sanitary napkin, etc.

The polysaccharide to be used is one capable of thickening in the presence of a polyvalent metal ion. It is required for the polysaccharide, upon contact with a small amount of a high viscous liquid (moisture), to be dissolved or dissociated and diffused quickly in the high viscous liquid thereby to fix the high viscous liquid. Accordingly, it is preferred for the polysaccharide to contain no polyvalent metal ions. Further, it is preferred for the thickening article of the present invention to contain no polyvalent metal ions. In the practice, however, cases are sometimes met with in which a trace amount of polyvalent metal ions enters unavoidably in the preparation of the polysaccharide, the production of the thickening article or the preparation of members constituting the thickening article. Hence, the situation in which the polysaccharide or the thickening article contains "no polyvalent metal ions" includes cases where they are completely free from either polyvalent metal ions or substances capable of generating polyvalent metal ions in water and cases where polyvalent metal ions or substances capable of generating polyvalent metal ions in water have been unavoidably incorporated in such small amounts that do not interfere with development of thickening to a degree enough to produce a leakproofing effect.

The polysaccharides which contain no polyvalent metal ions include polysaccharides in their free form not forming a metal salt and polysaccharides in their salt form with a monovalent metal ion. The polysaccharides which are not in a metal salt form include pectin, gellan gum, κ-carrageenan, glucomannan, guar gum, casein, and propylene glycol alginate. The polysaccharides in a salt form with a monovalent metal ion include sodium alginate. From the standpoint of cost and water solubility, preferred polysaccharides are pectin, sodium alginate, gellan gum, κ-carrageenan, and the like.

Since the polysaccharide positively dissolves and diffuses in a discharged liquid to thicken the liquid as described above, it is soluble in water or capable of being dissociated in water. The term "capable of being dissociated in water" as used herein means that the polysaccharide does not completely dissolve in water but is ready to be dispersed in water, i.e., it can exist in water with its molecular chains entangled. The term "completely dissolve in water" means that all the molecules of the polysaccharide are individually hydrated.

In the present invention the polysaccharide is required to react sensitively with a discharged liquid to thicken the liquid. Therefore, it is necessary that the polysaccharide should be present in the thickening article in a state ready to dissolve or be dissociated in water. This is totally different from the technique of WO95/17147 *supra* in which the polysaccharide is incorporated into the matrix of an absorbent article to fix liquid. The "state ready to dissolve or be dissociated in water" includes a state in which a powdered polysaccharide is dispersed in the absorbent member as shown in Figs. 1 and 2.

In order to adequately control the thickening rate of the polysaccharide, the physical properties of the gel formed by thickening, and the like, the degree of esterification of the polysaccharide can be adjusted, or the pH can be adjusted by addition of an organic acid, etc.

The polysaccharide can be used in a powdered form as noted above or in a particulate form. It can be encapsulated in water-soluble capsules. When used in the form of powder or particle, a preferred size is 10 to 1000 μm, particularly 50 to 500 μm. When used in the form of capsules, a preferred size of the capsules is 300 to 2000 μm, particularly 500 to 1000 μm.

The polysaccharide is preferably used in an amount of 10 to 200 $g/m^2$, particularly 30 to 100 $g/m^2$, from the standpoint of cost and the thickening ability (leakproofing effect). The weight ratio of the polysaccharide to the pulp fiber (polysaccharide/pulp fiber) is preferably 10/1000 to 200/50, particularly 1/5 to 1/1. The pulp fiber is preferably used in an amount of 50 to 1000 $g/m^2$, particularly 50 to 300 $g/m^2$.

Taking into consideration the balance with the amounts of the polysaccharide and the pulp fiber in the upper absorbent member 4a, the amount of the superabsorbent polymer in the lower absorbent member 4b is preferably 0 to 500 $g/m^2$, particularly 50 to 300 $g/m^2$.

While the thickening article of the present invention has been described with reference to its preferred embodiment the present invention is not construed as being limited thereto, and various changes and modifications can be made therein without departing from the spirit and scope thereof.

The polysaccharide can be disposed either on the entire area for liquid absorption or in a specific area of the diaper 1.

As far as the effects of the present invention are not impaired, the polysaccharide can be used in combination with an antimicrobials, antiseptics, or antifungals. The antimicrobials include benzethonium chloride and benzalkonium chloride. The antiseptics include parahydroxybenzoates (e.g., ethyl p-hydroxybenzoate and methyl p-hydroxybenzoate). Growth of microorganisms during storage can be prevented in the presence of these additives. pH Buffers, antiinflammatory agents, lipase inhibitors or urease inhibitors can also be used in combination to suppress a diaper rash caused particularly by soft feces.

The thickening article of the present invention is applicable to not only disposable diapers but other sanitary articles having an absorbent member, i.e., sanitary napkins, incontinence pads, nursing breast pads, etc.; sanitary articles having no absorbent member, i.e., diaper liners and panty liners; and surgical blood absorbent articles such as dressing; and the like.

EXAMPLE 1

A mixture of fluff pulp and pectin powder (particle size: 150 μm) was built up on absorbent paper (tissue) and enveloped in the absorbent paper to form an upper absorbent layer. The basis weight of the fluff pulp was 50 $g/m^2$, and that of the pectin powder was 50 $g/m^2$. Particles of a superabsorbent polymer were scattered on a polyethylene sheet as a backsheet to give a basis weight of 200 $g/m^2$ to form a lower absorbent layer. The upper absorbent layer was superposed on the lower absorbent layer, and a topsheet made of suction heat-bonded nonwoven fabric of polypropylene fiber was further superposed thereon. The backsheet and the topsheet were joined and fixed together at their periphery with elastically stretchable members interposed therebetween at the periphery to obtain a disposable diaper shown in Figs. 1 and 2. The disposable diaper (including the pectin powder) contained substantially no polyvalent metal ions.

EXAMPLE 2 (for reference)

A disposable diaper was obtained in the same manner as in Example 1, except that the pectin powder was not used in the upper absorbent layer but sprinkled between the upper absorbent layer and the topsheet in an amount of 50 g/m$^2$.

EXAMPLE 3 (for reference)

A disposable diaper was obtained in the same manner as in Example 1, except that the pectin powder was not used in the upper absorbent layer but sprinkled on the topsheet which had been sprinkled with water in an amount of 50 g/m$^2$ and fixed thereto by its own adhesion.

EXAMPLES 4 TO 6

A disposable diaper was obtained in the same manner as in Example 1, except for replacing the pectin used in Example 1 with sodium alginate (Example 4), gellan gum (Example 5) or κ-carrageenan (Example 6).

COMPARATIVE EXAMPLE 1

A disposable diaper was obtained in the same manner as in Example 1, except that particles of a superabsorbent polymer were incorporated into the upper absorbent layer in place of the pectin powder used in Example 1.

Evaluation of Performance:

In order to evaluate the performance of the disposable diapers obtained in Examples and Comparative Example, a leakage test was carried out in accordance with the following method. Further, the thickening effect of the polysaccharides used in Examples 1 and 4 to 6 was evaluated in a thickening test according to the following method. The results obtained are shown in Table 1.

Leakage Test:

Thirty diapers for each Example were used by each of ten babies under 12 months. The number of diapers which had a leak was divided by the total number of diapers used was taken as a leak ratio for evaluating leakproofness.

$$\text{Leak ratio (\%)} = [(\text{number of diaper having a leak})/(\text{total number of used diapers})] \times 100$$

Thickening Test:

Each of the polysaccharides used in Examples 1 and 4 to 6 was added to 500 ml of an aqueous calcium chloride solution having a calcium ion content of 0.1% by weight to become a concentration of 0.2% by weight. The kinematic viscosity (20°C) of the aqueous solution was measured with a B-type viscometer (Model B8M, supplied by Tokyo Keiki) before and after addition of the polysaccharide. The measurement after the addition of the polysaccharide was made after 30 seconds from the addition. Even if heterogeneous gelation took place, the measurement was carried out.

TABLE 1

| | Leakage Test Result (%) | Kinematic Viscosity (mm$^2$/s) | |
|---|---|---|---|
| | | Before Addition | After Addition |
| Example 1 | 1.7 | 30 | 82 |
| Example 2* | 2.3 | 30 | 82 |

(continued)

|  | Leakage Test Result (%) | Kinematic Viscosity (mm$^2$/s) | |
|---|---|---|---|
|  |  | Before Addition | After Addition |
| Example 3* | 1.3 | 30 | 82 |
| Example 4 | 2.0 | 16 | 96 |
| Example 5 | 1.7 | 10 | 70 |
| Example 6 | 1.7 | 25 | 90 |
| Comparative Example 1 | 6.3 | - | - |
| *: for reference | | | |

As is apparent from the results shown in Table 1, it is seen that the diapers of Examples which contained the polysaccharide (the articles of the present invention) exhibit higher leakproofness against a high viscous liquid than the diaper of Comparative Example which contained no polysaccharide.

Industrial Applicability:

The present invention provides a thickening article having high leakproofness against high viscous liquids such as soft feces and blood.

The thickening article of the present invention is useful as a disposable diaper or a diaper liner which is worn particularly by newborn babies or infants who get rid of soft feces frequently, a sanitary napkins, a panty liner, an incontinence pad, a nursing breast pad, a surgical blood absorbent article, and so forth.

## Claims

1.   An article for thickening body fluids or excreta comprising a liquid retentive absorbent member (4) which has a double-layered structure having an upper absorbent member (4a) and a lower absorbent member (4b) wherein a polysaccharide powder (10) capable of thickening in the presence of a polyvalent metal ion is present in the upper absorbent member (4a), in a state ready to dissolve or be dissociated in the moisture contained in body fluids or excreta, wherein said polysaccharide (10) contains no polyvalent metal ions, and wherein the lower absorbent member (4b) comprises pulp fiber (9) having superabsorbent polymer particles (12) dispersed therein or wherein the lower absorbent member (4b) consists solely of the superabsorbent polymer particles (12), wherein "upper" means facing to the wearer and wherein the upper absorbent member (4a) predominantly comprises pulp fiber (9) and the polysaccharide (10) is dispersed uniformly throughout the upper absorbent member (4a) in planar and thickness directions and they are wrapped in absorbent paper (11) to form the upper absorbent member (4a).

2.   The article for thickening body fluids or excreta as claimed In claim 1, wherein said polysaccharide (10) is present in a weight ratio of polysaccharide to pulp fiber of 10/1000 to 200/50.

3.   The article for thickening body fluids or excreta as claimed in claim 1, wherein said polysaccharide comprises pectin, gellan gum, κ-carrageenan, glucomannan, guar gum, sodium alginate or propylene glycol alginate.

## Patentansprüche

1.   Ein Gegenstand zum Eindicken von Körperflüssigkeiten oder Ausscheidungsstoffen, umfassend ein Flüssigkeit zurückhaltendes absorbierendes Element (4), das eine Doppelschichtstruktur mit einem oberen absorbierenden Element (4a) und einem unteren absorbierenden Element (4b) aufweist, wobei ein Polysaccharidpulver (10), das zur Eindickung in Gegenwart eines mehrwertigen Metallions in der Lage ist, in dem oberen absorbierenden Element (4a) in einem in der in Körperflüssigkeiten oder Ausscheidungsstoffen enthaltenen Feuchtigkeit lösungsbereiten oder dissoziierbaren Zustand vorliegt, wobei das Polysaccharid (10) keine mehrwertigen Metallionen enthält und wobei das untere absorbierende Element (4b) Zellstofffaser (9) mit darin dispergierten superabsorbierenden Polymerteilchen (12) umfasst oder wobei das untere absorbierende Element (4b) nur aus den superabsorbierenden Polymerteilchen (12) besteht, wobei "oberes" dem Träger zugewandt bedeutet und wobei das obere absorbierende Element (4a) vorwiegend Zellstofffaser (9) umfasst und das Polysaccharid (10) gleichförmig in dem oberen absorbierenden Element (4a) in Flächen- und Dickenrichtung dispergiert ist und diese in absorbierendes Papier (11) gehüllt sind, um das obere absorbierende Element (4a) zu bilden.

2. Der Gegenstand zum Eindicken von Körperflüssigkeiten oder Ausscheidungsstoffen nach Anspruch 1, wobei das Polysaccharid (10) in einem Gewichtsverhältnis von Polysaccharid zu Zellstofffaser von 10/1000 bis 200/50 vorliegt.

3. Der Gegenstand zum Eindicken von Körperflüssigkeiten oder Ausscheidungsstoffen nach Anspruch 1, wobei das Polysaccharid Pectin, Gellangummi, κ-Carrageenan, Glucomannan, Guarmehl, Natriumalginat oder Propylenglykolalginat umfasst.

## Revendications

1. Article pour épaissir des fluides corporels ou des excréments comprenant un élément absorbant rétenteur de liquides (4) qui a une structure à double couche comportant un élément absorbant supérieur (4a) et un élément absorbant inférieur (4b), dans lequel une poudre de polysaccharide (10) capable d'épaississement en présence d'un ion métal polyvalent est présente dans l'élément absorbant supérieur (4a), dans un état prêt à être dissous ou dissocié dans l'humidité contenue dans les fluides corporels ou les excréments, dans lequel ledit polysaccharide (10) ne contient pas d'ions métal polyvalent, et dans lequel l'élément absorbant inférieur (4b) comprend une fibre à base de pulpe (9) contenant des particules polymères superabsorbantes (12) à l'état dispersé ou dans lequel l'élément absorbant inférieur (4b) est uniquement constitué par les particules polymères superabsorbantes (12), dans lequel "supérieur" signifie tourné vers le porteur et dans lequel l'élément absorbant supérieur (4a) essentiellement comprend de la fibre à base de pulpe (9) et le polysaccharide (10) est dispersé de manière uniforme dans l'élément absorbant supérieur (4a) dans les directions planaire et d'épaisseur et ceux-ci sont emballés dans un papier absorbant (11) de manière à former l'élément absorbant supérieur (4a).

2. Article pour épaissir des fluides corporels ou des excréments selon la revendication 1, dans lequel ledit polysaccharide (10) est présent dans un rapport en poids de polysaccharide sur fibre à base de pulpe de 10/1000 à 200/50.

3. Article pour épaissir des fluides corporels ou des excréments selon la revendication 1, dans lequel ledit polysaccharide comprend la pectine, la gomme gellane, la κ-carraghénine, le glucomannane, la gomme de guar, l'alginate de sodium ou l'alginate de propylène glycol.

Fig. 1

Fig. 2

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9517147 A